# EUROPÄISCHE PATENTSCHRIFT

(11) **EP 1 212 015 B1**
(45) Veröffentlichungstag und Bekanntmachung des Hinweises auf die Patenterteilung: **21.02.2007**
(21) Anmeldenummer: 99974009.5
(22) Anmeldetag: 14.09.1999
(51) Int. Cl.: A61F 2/44, A61F 2/46

(54) **EINSETZINSTRUMENT FÜR EIN ZWISCHENWIRBELIMPLANTAT**
INSTRUMENT FOR INSERTING INTERVERTEBRAL IMPLANTS
INSTRUMENT D'INSERTION POUR IMPLANT INTERVERTEBRAL

(43) Veröffentlichungstag der Anmeldung: 12.06.2002
(73) Patentinhaber: Spine Solutions Inc., New York, NY 10022 (US)
(72) Erfinder: BEYERSDORFF, Boris, D-78532 Tuttlingen (DE); MARNAY, Thierry, F-34080 Montpellier (FR)
(74) Vertreter: Jochem, Bernd
(86) Internationale Anmeldenummer: PCT/EP1999/006803
(87) Internationale Veröffentlichungsnummer: WO 2001/019295

(56) Entgegenhaltungen:
- EP-A- 0 333 990
- EP-A- 0 471 821
- DE-A- 4 328 690
- DE-U- 29 916 078
- FR-A- 2 737 656

## Beschreibung

Die Erfindung betrifft ein Einsetzinstrument für ein dreiteiliges Zwischenwirbelimplantat, welches ein aneinem Wirbelkörper anlegbares Oberteil, ein am benachbarten Wirbelkörper anlegbares Unterteil und ein dazwischen einsetzbares Gelenkelement umfaßt, mit zwei an einem Ende gegeneinander schwenkbar gelagerten, nebeneinander angeordneten Armen, die an ihrem freien Ende je eine Halteeinrichtung für das Oberteil beziehungsweise das Unterteil des Zwischenwirbelimplantats aufweisen.

Ein solches Einsetzinstrument ist beispielsweise aus der EP 0 471 821 B1 und der EP 0 333 990 A1 bekannt. Das Einsetzinstrument ist zangenartig ausgebildet und kann auch dazu dienen, nach dem Einsetzen von Oberteil und Unterteil des Zwischenwirbelimplantats die beiden Wirbelkörper voneinander zu entfernen, um dadurch Raum zur Einführung des Gelenkelements zu erhalten. Dieses Gelenkelement muß bei dem bekannten Instrument mit anderen Instrumenten in den Zwischenraum zwischen Oberteil und Unterteil des Zwischenwirbelimplantats eingeführt werden. Dies ist ein schwieriger Vorgang, bei dem die Gefahr besteht, daß das Gelenkteil gegenüber den beiden anderen Implantatteilen verkantet eingeführt und dadurch beschädigt wird.

Es ist auch bekannt, zum Einsetzen von vollständigen Zwischenwirbelimplantaten diese in einer Längsführung bis an die Implantatstelle heranzuführen und dann aus der Führung in den Zwischenwirbelraum abzugeben (DE 43 28 690). Ein solches Instrument ist nur für das Einsetzen vollständiger Zwischenwirbelimplantate geeignet, außerdem ergibt sich das Problem einer genauen Justierung dieser Führung relativ zu dem Zwischenwirbelraum, bei Fehljustierungen kann es zu verkantetem Einschub des Zwischenwirbelimplantats kommen, und dies kann zu Verletzungen führen.

Es ist Aufgabe der Erfindung, ein gattungsgemäßes Einsetzinstrument so auszubilden, daß diese Nachteile vermieden werden und die Einführung des Gelenkelements vereinfacht wird.

Diese Aufgabe wird bei einem Einsetzinstrument der eingangs beschriebenen Art erfindungsgemäß dadurch gelöst, daß in einem der Arme eine Längsführung für das Gelenkelement angeordnet ist.

Man erhält dadurch ein kombiniertes Einsetzinstrument, welches zunächst der Handhabung von Oberteil und Unterteil des Implantats dient und mit dem Oberteil und Unterteil in der gewünschten Position in den Zwischenwirbelraum einbringbar sind. Durch die verschwenkbare Lagerung der Arme können Oberteil und Unterteil dann unter Aufweitung des Zwischenwirbelraums in an sich bekannter Weise von einander entfernt werden, so daß ein Einführraum für das Gelenkelement zwischen ihnen entsteht. In diesen Einführraum wird das Gelenkelement dann über die Führung in einem der beiden Arme des Einsetzinstruments direkt eingeschoben, wobei durch die Verbindung der beiden Arme des Einsetzinstruments mit den in den Zwischenwirbelraum eingesetzten Teilen des Implantats eine zuverlässige Justierung der Längsführung für das Gelenkelement gewährleistet ist, außerdem ist sichergestellt, daß das Gelenkelement exakt in der gewünschten Relativposition zu den anderen beiden Implantatteilen in den Zwischenwirbelraum eingeführt wird.

Sowohl das Einsetzen von Oberteil und Unterteil des Implantats als auch das Einführen des Gelenkelements kann somit mit einem einzigen Instrument erfolgen, ein Absetzen und Auswechseln ist nicht mehr notwendig, dieses Einsetzinstrument übernimmt eine größere Anzahl von Funktionen, nämlich die des Einsetzens von Oberteil und Unterteil des Zwischenwirbelimplantats, die der Aufweitung des Zwischenwirbelraums und schließlich die der Einführung des Gelenkelements in den Zwischenraum zwischen Oberteil und Unterteil des Implantats.

Günstig ist es, wenn die Längsführung durch in Längsnuten eingreifende Vorsprünge gebildet wird.

Beispielsweise kann vorgesehen sein, daß in einem der Arme in einem in dessen Längsrichtung verlaufendem Aufnahmeraum für das Gelenkelement einander gegenüberliegende Nuten angeordnet sind, in die das Gelenkelement mit seitlichen Vorsprüngen eingreift.
Bei einer besonders bevorzugten Ausführungsform ist vorgesehen, daß der die Längsführung umfassende Arm zwei im Abstand und parallel zueinander angeordnetem stabförmige Schenkel aufweist, die zwischen sich einen Aufnahmeraum für das Gelenkelement bilden und dieses längs des Aufnahmeraums zwischen sich führen.

Günstig ist es, wenn die Längsführung an ihrem der Schwenklagerung der Arme benachbarten Ende einen Einsetzbereich bildet, an dem das Gelenkelement in die Längsführung einschiebbar ist. Dieser Einsetzbereich kann beispielsweise dadurch erfolgen, daß Längsnuten stirnseitig offen sind, in einem anderen Ausführungsbeispiel kann vorgesehen sein, daß die Längsführung erst in einem Abstand von der Schwenklagerung beginnt, der der Länge des einzusetzenden Gelenkelements entspricht.

Bei einer besonders bevorzugten Ausführungsform ist vorgesehen, daß die Längsführung des einen Armes in eine Längsführung des an dem Arm gehaltenen Teils des Zwischenwirbelimplantats übergeht. Man erhält dadurch eine kontinuierliche Längsführung für das Gelenkelement einmal längs des Armes und zum anderen auch längs des einen Teils des Zwischenwirbelimplantats, so daß eine absolut präzise Einführung des Gelenkelements in das angeschlossene Teil des Zwischenwirbelimplantats gewährleistet ist. Dieses an den Arm angeschlossene Teil des Implantats bildet während des Einschubvorgangs praktisch ein Teil des Einsetzinstrument, das nach dem Einführen des Gelenkelements vom Einsetzinstrument abgetrennt wird und als Implantatteil im Zwischenwirbelraum verbleibt.

Bei einer weiteren bevorzugten Ausführungsform ist vorgesehen, daß das Einsetzinstrument einen in die Längsführung einsetzbaren Vorschubkörper umfaßt, der mit einem stabförmigen Schubelement verbunden ist. Damit kann das Gelenkelement längs der Längsführung bis in den Zwischenwirbelraum vorgeschoben werden.
Es ist besonders vorteilhaft, wenn die beiden Arme gemäß einer bevorzugten Ausführungsform der Erfindung an ihren freien Enden derart nebeneinander angeordnet sind, daß die Halteeinrichtungen sich in Richtung der Schwenkbewegung der Arme zumindest teilweise überlappen. Man kann dadurch eine sehr geringe Bauhöhe des Einsetzinstruments realisieren, diese Bauhöhe liegt in der Größenordnung der Spaltbreite des Zwischenwirbelraums, und es ist außerdem möglich, dadurch die beiden Teile des Implantats, die mit den Armen des Einsetzinstruments verbunden werden, ebenfalls sehr dicht aneinander zu führen und dadurch eine sehr geringe Bauhöhe zu realisieren. Auf diese Weise können diese beiden Teile des Implantats ohne große Aufweitung des Zwischenwirbelraums in diesen eingeführt werden, die Aufweitung des Zwischenwirbelraums erfolgt erst nach der Einführung dieser Teile des Zwischenwirbelimplantats durch Auseinanderschwenken der diese beiden Teile des Implantats haltenden Arme.

Es ist vorteilhaft, wenn die Schwenklagerungen der beiden Arme einen Abstand voneinander haben, so daß die Arme in einer Einsetzstellung des Oberteils und des Unterteils des Zwischenwirbelimplantats, bei der die freien Enden der Arme einander maximal angenähert sind, am gelagerten Ende einen größeren Abstand voneinander haben als am freien Ende. Auch dies trägt dazu bei, die Bauhöhe des Einsetzinstruments und der daran gehaltenen Implantatteile beim Einsetzen so gering wie möglich zu gestalten.

Außerdem ist es bei dieser Anordnung gemäß einer bevorzugten Ausführungsform möglich, daß ein Spreizelement vorgesehen ist, das sich an beiden Armen abstützt und längs der Arme in Richtung auf das freie Ende der Arme vorschiebbar ist und dabei die Arme auseinander schwenkt. Damit ist allein durch Vorschieben des Spreizelements längs der Arme das Aufweiten des Zwischenwirbelraums möglich, nachdem Oberteil und Unterteil des Zwischenwirbelimplantats in den Zwischenwirbelraum eingesetzt sind.

Dabei ist es günstig, wenn mindestens einer der beiden Arme eine Längsführung für den Spreizkörper aufweist, so daß dieser längs der Arme definiert geführt wird.

Außerdem kann am Spreizkörper eine Vorschubstange angeordnet sein, mit dessen Hilfe der Spreizkörper längs der Arme verschoben wird.

Bei einer besonders bevorzugten Ausführungsform ist dabei vorgesehen, daß die Vorschubstange als Zahnstange ausgebildet ist, die mit einem Antriebszahnrad im Bereich der Schwenklagerung der Arme kämmt, dadurch ist eine sehr gefühlvolle Vorschubbewegung des Spreizkörpers längs der Arme möglich, wobei auch große Kräfte über die Zahnverbindung übertragen werden können.

Die Haltevorrichtungen, mit denen die Implantatteile an den Armen gehalten sind, können sehr unterschiedlich ausgebildet sein, besonders bevorzugt wird eine Ausgestaltung, bei der die Haltevorrichtungen Stifte sind, die in Öffnungen des Oberteiles beziehungsweise des Unterteils des Zwischenwirbelimplantats eingreifen.

Bei einer bevorzugten Ausführungsform ist vorgesehen, daß die Haltevorrichtungen zumindest an einem der Arme um eine Schwenkachse verschwenkbar sind, die im Bereich des freien Endes des Armes angeordnet ist und parallel zur Schwenkachse des Armes verläuft, und daß die Haltevorrichtungen nach dem Verschwenken um diese Schwenkachse in unterschiedlichen Winkelstellungen feststellbar sind. Dadurch ist es möglich, die Neigung der beiden Implantatteile relativ zu einander geringfügig zu verändern, beispielsweise im Bereich von 1° bis 5°, so daß neben der Implantathöhe auch der Implantatwinkel so gewählt werden kann, wie es der korrekten Positionierung der Wirbelkörper entspricht.

Bei einer bevorzugten Ausführungsform kann dabei zur Feststellung der Winkelstellung ein Fixierstift vorgesehen sein, der in bei unterschiedlicher Winkelstellung miteinander ausgerichtete Bohrungen einschiebbar ist.

Gemäß einer weiteren bevorzugten Ausführungsform weist mindestens eine Halteeinrichtung eine lösbare Verriegelung auf. Durch diese lösbare Verriegelung wird das an dem Arm gehaltene Implantatteil unlösbar mit dem Arm verbunden, erst nach Entriegelung dieser Verriegelung ist es möglich, Implantatteil und Einsetzinstrument wieder zu trennen.

Dadurch wird eine unbeabsichtigte Trennung des Einsetzinstruments von den Implantatteilen vermieden, es ist sogar möglich, auf diese Weise bereits implantierte Implantatteile wieder aus dem Zwischenwirbelraum herauszuziehen, falls dies notwendig werden sollte.

Günstig ist es dabei, wenn die Verriegelung durch eine Verdrehung eines Riegels um eine Drehachse erfolgt, die parallel zu Längsachse des Armes verläuft, an dem die Halteeinrichtung angeordnet ist.

Insbesondere kann bei einer bevorzugten Ausführungsform vorgesehen sein, daß der die Halteeinrichtung tragende Arm oder ein Teil desselben um seine Längsachse verdrehbar ist und einen Riegel trägt, der in einer Stellung das an der Halteeinrichtung gehaltene Teils des Zwischenwirbelimplantats unlösbar mit diesem verriegelt und in einer anderen Stellung freigibt.

Eine besonders vorteilhafte Ausgestaltung ergibt sich, wenn die Halteeinrichtung ein in eine Aufnahmebohrung am gehaltenen Teil des Zwischenwirbelimplantats eingreifender Stift und der Riegel ein seitlich von diesem abstehender Vorsprung ist, der in einer Winkelstellung des Stifts in einen entsprechenden Rücksprung des Implantatteils eingreift, in einer anderen Winkelstellung jedoch aus diesem Rücksprung austritt.

Bei einer besonders bevorzugten Ausführungsform ist vorgesehen, daß der die Längsführung aufweisende Arm zwei parallel zueinander angeordnete Schenkel aufweist, die zwischen sich einen Aufnahmeraum für das Gelenkelement ausbilden, und daß der andere Arm mittig zwischen diesen Schenkeln verläuft, so daß er mit seinem freien Ende zwischen die Schenkel eintauchen kann.

Weiterhin kann vorgesehen sein, daß ein zwischen den Armen angeordnetes, längs derselben verschiebliches Spreizelement auf der Oberseite der beiden Schenkel aufliegt und mit einem Vorsprung zwischen die beiden Schenkel in den Aufnahmeraum eingreift. Dadurch ergibt sich eine Führung des Spreizelements längs der Arme.

Zusätzlich kann das Spreizelement an seiner Oberseite eine Vertiefung aufweisen, in die der andere Arm eintaucht. Auch diese trägt zur Führung des Spreizelements bei.

Die Schenkel des einen Armes können im Querschnitt rechteckig sein, der andere Arm kann im Querschnitt kreisförmig sein.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht des Einsetzinstruments nach dem Einführen des Oberteils und des Unterteils eines Zwischenwirbelimplantats in den Zwischenwirbelraum, vor dem Aufspreizen des Zwischenwirbelraums und vor dem Einführen des Gelenkelements in den Zwischenwirbelraum;
- Figur 2:: eine Draufsicht auf den oberen Arm des Einsetzinstruments der Figur 1 mit an ihm gehaltenem Oberteil des Zwischenwirbelimplantats;
- Figur 3:: eine Seitenansicht in Richtung des Pfeiles A in Figur 2;
- Figur 4:: eine Seitenansicht des unteren Armes mit daran gehaltenem Unterteil des Zwischenwirbelimplantats, welches längs Linie 4-4 in Figur 5 geschnitten dargestellt ist;
- Figur 5:: eine Draufsicht auf den unteren Arm in Richtung des Pfeiles B in Figur 4;
- Figur 6:: eine perspektivische Ansicht des Einsetzinstruments mit daran gehaltenem Oberteil und Unterteil in der Einsetzposition mit maximal angenäherten Implantatteilen;
- Figur 7:: eine perspektivische Ansicht des Einsetzinstruments der Figur 6 nach dem Einsetzen des Oberteils und des Unterteils des Zwischenwirbelimplantats in den Zwischenwirbelraum und nach dem Aufweiten desselben kurz vor dem Einschieben des Gelenkelements zwischen Oberteil und Unterteil des Zwischenwirbelimplantats;
- Figur 8:: eine Seitenansicht des vorderen Teils des Einsetzinstruments der Figur 7 kurz vor dem Einschieben des Gelenkelements zwischen Oberteil und Unterteil des Zwischenwirbelimplantats;
- Figur 9:: eine Schnittansicht längs Linie 9-9 in Figur 8 und
- Figur 10:: eine Ansicht ähnlich Figur 8 nach dem Einschieben des Gelenkelements zwischen Oberteil und Unterteil des Zwischenwirbelimplantats.

Das in der Zeichnung dargestellte Einsetzinstrument 1 dient dem Einsetzen eines Zwischenwirbelimplantats 2 in den von zwei Wirbelkörpern 3, 4 begrenzten Zwischenwirbelraum 5.

Das Zwischenwirbelimplantat 2 umfaßt dabei ein im wesentlichen plattenförmiges Oberteil 6 mit einer oberen ebenen Anlagefläche 7 und von dieser abstehenden Verankerungselementen 8 sowie ein ebenfalls plattenförmiges Unterteil 9 mit einer ebenen äußeren Anlagefläche 10 und von dieser abstehenden Verankerungselementen 11.

Das Oberteil 6 weist an seiner dem Unterteil 9 zugewandten Seite eine kugelkalottenförmige Lagerfläche 12 auf, in das Unterteil 9 ist eine Vertiefung 13 eingearbeitet, die zu einer Seite hin offen ist und die einen Einschubraum für ein ebenfalls Teil des Zwischenwirbelimplantats 2 bildendes Gelenkelement 14 bildet. Dieses Gelenkelement 14 weist einen plattenförmigen im wesentlichen rechteckigen Sockel 15 und einen einseitig von diesem zentral abstehenden Lagervorsprung 16 auf, dessen Oberseite eine kugelkalottenförmige Lagerfläche 17 ausbildet.

Das Gelenkelement 14 kann von der offenen Seite in die Vertiefung 13 eingeschoben werden, wobei die seitlichen Ränder des Sockels 15 in seitliche Nuten 18 im Unterteil 9 eingreifen, so daß das Gelenkelement 14 längs dieser Nuten 18 geführt in die Vertiefung 13 einschiebbar ist.
Im implantierten Zustand greift die Lagerfläche 17 in die konkave Lagerfläche 12 des Oberteils ein, so daß Oberteil 6 und Unterteil 9 sich über das Gelenkelement gegeneinander abstützen und gegeneinander verschwenkbar sind.

Sowohl das Oberteil 6 als auch das Unterteil 9 weisen an einer Seitenfläche im wesentlichen parallel zu den Anlagefläche 7 beziehungsweise 10 verlaufende Einstecköffnungen 19 auf, in die Haltestifte 20 des Einsetzinstruments 1 einsteckbar sind.

Dieses Einsetzinstrument 1 weist einen ersten länglichen Arm 21 mit zwei im Abstand zueinander und parallel zueinander verlaufenden Schenkeln 22, 23 auf, die einseitig um ihre Längsachse verdrehbar an einem Lagerbock 24 gehalten sind. Beide Schenkel 22 und 23 weisen einen quadratischen Querschnitt auf und bilden stangenförmige, lange Elemente, die an ihrem freien Ende in Verlängerung der Drehachse der Schenkel jeweils einen der Haltestifte 20 tragen.

An diesen Haltestiften 20 der Schenkel 22 und 23 sind außerdem radial abstehende Riegelvorsprünge 25 vorgesehen, die beispielsweise durch in die Haltestifte 20 radial eingesteckte Stifte realisiert werden können, die in einer Winkelstellung der Schenkel 22 in seitliche Rücksprünge 26 des Unterteils 9 eingreifen, diese Rücksprünge 26 sind zum Oberteil 6 hin offen, so daß durch Verdrehung der Schenkel 22 und 23 um 90° die Riegelvorsprünge 25 so verdreht werden können, daß sie aus den Rücksprüngen 26 austreten. Solange die Riegelvorsprünge 25 in die Rücksprünge 26 eingreifen, sind die Schenkel 22 und 23 bei in die Einstecköffnungen 19 eingesteckten Haltestiften 20 lösbar mit dem Unterteil 9 verbunden, werden die Riegelvorsprünge 25 jedoch durch Verdrehung der Schenkel 22 und 23 aus den Rücksprüngen 26 herausgedreht, können die Haltestifte 20 aus den Einstecköffnungen 19 herausgezogen werden, so daß eine Verschiebung der Schenkel 22 und 23 gegenüber dem Unterteil 9 möglich wird und damit ein Einschieben oder Trennen.

Die Schenkel 22 und 23 können durch eine in der Zeichnung nicht dargestellte Rastung in den Endstellungen lösbar festgelegt werden, in denen der Riegelvorsprung 25 in den Rücksprung 26 eingreift beziehungsweise in denen er vollständig aus dem Rücksprung 26 austritt.

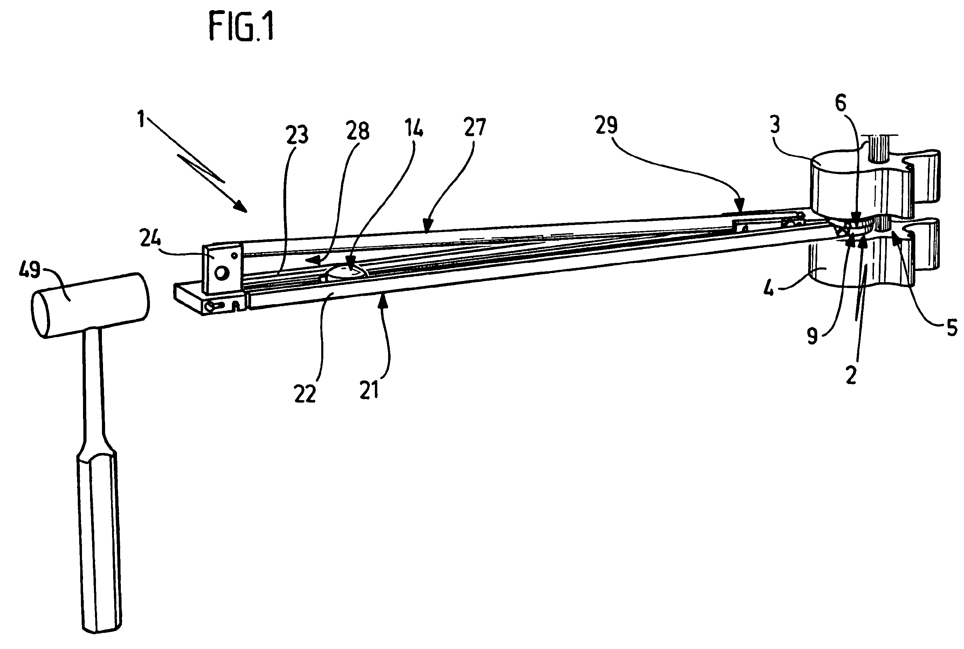

An dem Lagerbock 24 ist im Abstand von der Ebene, die durch die beiden Schenkel 22 und 23 aufgespannt wird, ein zweiter Arm 27 um eine Drehachse verschwenkbar gelagert, die quer zur Längsrichtung der Schenkel 22 und 23 und parallel zu der von diesen aufgespannten Ebene verläuft, der Arm 27 ist dabei etwa in der Mitte zwischen den beiden Schenkeln 22 und 23 angeordnet, so daß das freie Ende des Arms 27 in den Zwischenraum 28 zwischen den beiden Schenkeln 22 und 23 eintreten kann. Aufgrund des Abstandes der Lagerstelle der Armes 27 von der durch die Schenkel 22 und 23 aufgespannten Ebene nimmt dabei der Abstand der Armes 27 vom Arm 21 kontinuierlich ab, wie dies aus der Darstellung der Figur 1 deutlich wird.

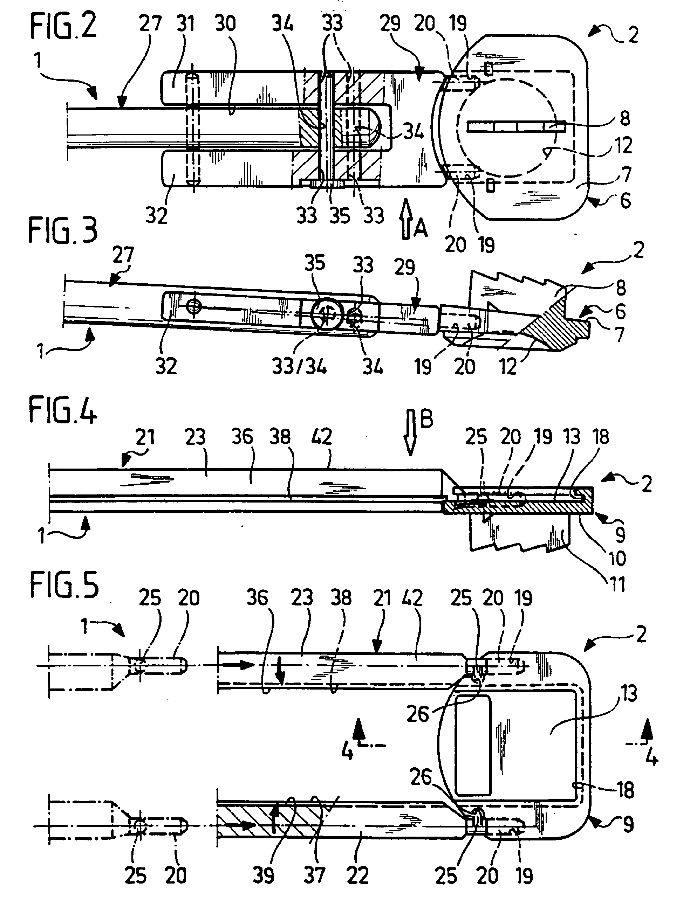

Der Arm 27 ist im Querschnitt kreisförmig ausgebildet und trägt an seinem freien Ende eine U-förmigen Halter 29, der das freie Ende des Armes 27 im Zwischenraum 30 zwischen zwei parallel verlaufenden Schenkeln 31, 32 aufnimmt. Im Bereich der freien Ende der Schenkel 31, 32 sind der Halter 29 und der Arm 27 um eine parallel zur Schwenkachse des Armes 27 verlaufende Drehachse verschwenkbar miteinander verbunden. Dadurch kann der Halter 29 relativ zum Arm 27 unterschiedliche Winkelstellungen einnehmen, in Figur 3 sind zwei sich um einen kleinen Winkelbetrag unterscheidende Winkelstellungen mit strichpunktierten Linien eingetragen. Zur Festlegung des Halters 29 in unterschiedlichen Winkelstellungen sind sowohl in den Schenkeln 31 und 32 als auch im Arm 27 Querbohrungen 33 beziehungsweise 34 vorgesehen, und zwar in Längsrichtung versetzt mehrere derartige Paare von Querbohrungen, die bei unterschiedlichen Stellungen des Halters 29 relativ zum Arm 27 miteinander ausgerichtet sind. In diese Paare von Querbohrungen 33, 34 kann ein Fixierstift 35 eingeschoben werden. Da die zusammengehörenden Querbohrungen 33, 34 bei den verschiedenen Paaren eine unterschiedliche Position einnehmen, ergibt sich für jedes Paar von Querbohrungen beim Einsetzen eines Fixierstifts 35 eine unterschiedliche Winkellage relativ zum Arm 27, die Schwenkwinkel liegen dabei in der Größenordnung einiger Grad, beispielsweise wird insgesamt ein Bereich überstrichen, der zwischen 1° und 5° liegen kann.

An dem Halter 29 sind Haltestifte 20 angeordnet, die in der beschriebenen Weise in Einstecköffnungen 19 des Oberteils 6 einschiebbar sind. Durch die unterschiedliche Winkelstellung des Halters 29 ist es möglich, das Oberteil 6 gegenüber dem Unterteil 9, das an den Schenkeln 22 und 23 gehalten ist, in geringem Umfang zu verkippen.

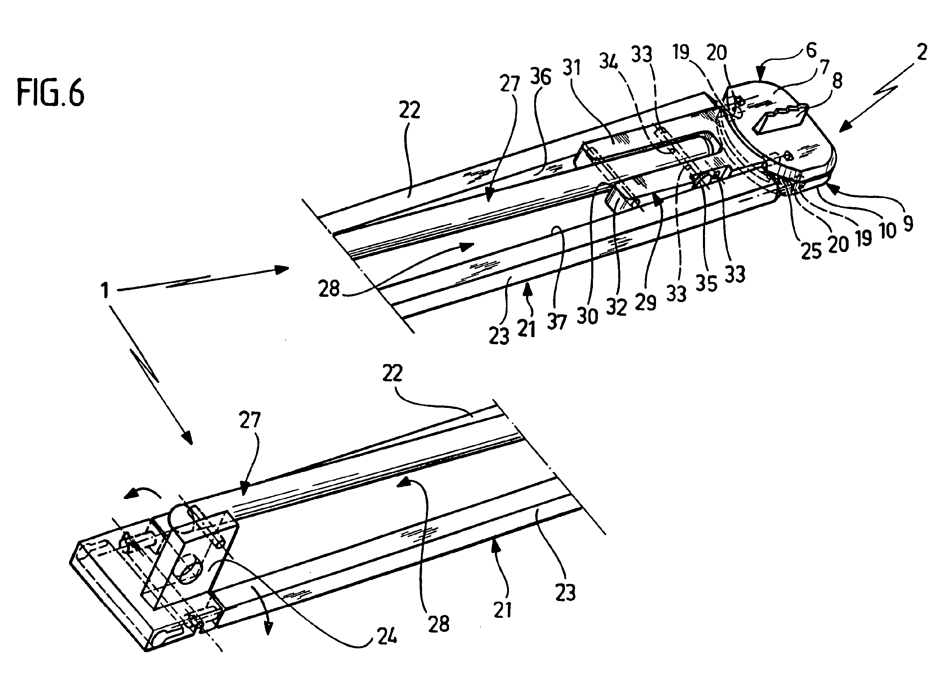

Die Breite des Halters 29 ist so gewählt, daß der Halter 29 in den Zwischenraum 28 zwischen den beiden Schenkeln 22 und 23 hineinpaßt, so daß die Haltestifte 20 am Halter 29 und an den Schenkeln 22 und 23 praktisch nebeneinander angeordnet werden können, dadurch ist es möglich, Oberteil 6 und Unterteil 9 in einer maximal angenäherten Position an den beiden Armen 21 und 27 zu halten, diese Position wird als Einsetzposition bezeichnet (Figuren 1 und 6).

Die beiden Schenkel 22 und 23 tragen in den Innenflächen 36, 37, die einander zugewandt sind, wenn die Riegelvorsprünge 25 in die Rücksprünge 26 eingreifen, einander gegenüberliegende Längsnuten 38, 39, die eine Längsführung für das Gelenkelement 14 ausbilden. Die Dimensionierung dieser Längsnuten 38 und 39 entspricht der der Seitenränder des Sockels 15 des Gelenkelements 14, so daß das Gelenkelement 14 in dem Zwischenraum 28 zwischen den Schenkeln 22 und 23 in Längsrichtung geführt wird, wenn die Seitenränder des Sockels 15 in die Längsnuten 38 und 39 eintauchen. Diese Längsnuten 38 und 39 enden in einem Abstand vor dem Lagerbock 24, der ein Einsetzen des Sockels 15 in die Längsnuten 38, 39 ermöglicht, und diese Längsnuten 38 und 39 setzen sich bis an das freie Ende der Schenkel 22 und 23 fort, dort gehen sie unmittelbar über in die beidseitig der Vertiefung 13 angeordneten Nuten 18, die der Aufnahme des Sockels 15 dienen. Man erhält damit eine von den Schenkeln 22 und 23 direkt bis in das Unterteil 9 des Zwischenwirbelimplantats 2 führende Führungsbahn für das Gelenkelement 14.

In die Längsnuten 38 und 39 ist außerdem ein plattenförmiger Vorschubkörper 40 einsetzbar, der gelenkig mit einer Schubstange 41 verbunden ist. Mittels dieser Schubstange 41 läßt sich das in die Längsnuten 38 und 39 eingesetzte Gelenkelement 14 längs seiner Führungsbahn vorschieben, der Vorschubkörper 40 wird dazu nach dem Gelenkelement 14 in die durch die Längsnuten 38 und 39 gebildete Führungsbahn eingeführt.

Auf der ebenen Oberseite 42 der Schenkel 22 und 23 stützt sich ein den Zwischenraum 28 zwischen den beiden Schenkeln 22 und 23 überbrückendes Spreizelement 43 ab, welches mit einem Vorsprung 44 in den Zwischenraum 28 geringfügig eintaucht und dadurch quer zur Längsrichtung der Schenkel 22 und 23 geführt wird. Dieses Spreizelement 43 weist an seinem den Schenkeln 22 und 23 abgewandten Ende eine Vertiefung 45 mit kreisbogenförmigem Querschnitt auf, in die der Arm 27 eintaucht. Das Spreizelement 23 ist mit einer als Zahnstange ausgebildeten Schubstange 46 verbunden, diese kämmt mit einem Zahnrad 47, welches am Lagerbock 24 verdrehbar gelagert ist und mittels eines Griffteil 48 verdreht werden kann. Bei dieser Verdrehung wird die Schubstange 46 verschoben, und dies führt zu einer Längsverschiebung des Spreizelements 43 längs der Schenkel 22 und 23. Beim Vorschieben des Spreizelements 43 wird dadurch der Arm 27 von den Schenkeln 22 und 23 weggeschwenkt, die Arme 27 und 21 werden also gespreizt, so daß dadurch das Oberteil 6 und das Unterteil 9 voneinander entfernt werden. Dies führt auch zu einem Auseinanderdrücken der Wirbelkörper 3 und 4 und damit zu einer Aufweitung des Zwischenwirbelraums 5.

Das beschriebene Einsetzinstrument besteht vorzugsweise aus einem körperverträglichen Metall, beispielsweise aus Titan oder einer Titanlegierung, dasselbe gilt hinsichtlich des Oberteils 6 und des Unterteils 9 des Zwischenwirbelimplantats 2. Das Gelenkelement 14 wird aus einem körperverträglichen Kunststoffmaterial hergestellt, beispielsweise aus Polyethylen, und auch das Spreizelement 43 besteht vorzugsweise aus einem Kunststoffmaterial, um ein gutes Gleiten gegenüber den Schenkeln 22 und 23 und gegenüber dem Arm 27 zu gewährleisten.

Zum Einsetzen des Zwischenwirbelimplantats 2 in einen Zwischenwirbelraum 5 wird zunächst nach Entfernung der Bandscheibe aus dem Zwischenwirbelraum 5 dieser in geeigneter Weise vorbereitet, beispielsweise können senkrechte Nuten in die Wirbelkörper 3, 4 eingeschlagen werden, die die Verankerungselemente 8 beziehungsweise 11 des Zwischenwirbelimplantats 2 aufnehmen.

Nach entsprechender Vorbereitung werden das Oberteil 6 und das Unterteil 9 auf die Arme 27 beziehungsweise 21 aufgesteckt, das Unterteil 9 wird durch Verdrehung der Schenkel 22, 23 mit dem Arm 21 verriegelt, wobei die Riegelvorsprünge 25 in die Rücksprünge 26 des Unterteils 9 eingreifen, und die beiden Arme 21 und 27 werden in die Einsetzposition verschwenkt, bei der das Oberteil 6 und das Unterteil 9 einander maximal angenähert sind, diese beiden Teile haben also eine sehr geringe Bauhöhe. In dieser Einsetzposition werden Oberteil 6 und Unterteil 9 in den vorbereiteten Zwischenwirbelraum 5 eingeführt, beispielsweise durch Einschlagen mittels eines hammerähnlichen Instruments 49 (Figur 1). Dabei kann die Neigung, die das Oberteil 6 gegenüber dem Unterteil 9 einnimmt, durch Verschwenken des Halters 29 gegenüber dem Arm 27 vorgewählt werden, in der gewünschten Position wird die Winkelstellung durch den Fixierstift 35 fixiert.

Nach diesem Einsetzen von Oberteil 6 und Unterteil 9 werden nacheinander das Gelenkelement 14 und der Vorschubkörper 40 in die durch die Längsnuten 38, 39 gebildete Führungsbahn eingeschoben, außerdem werden der Vorschubkörper 40 sowie die Schubstange 41 und das Zahnrand 47 in das Instrument eingesetzt.

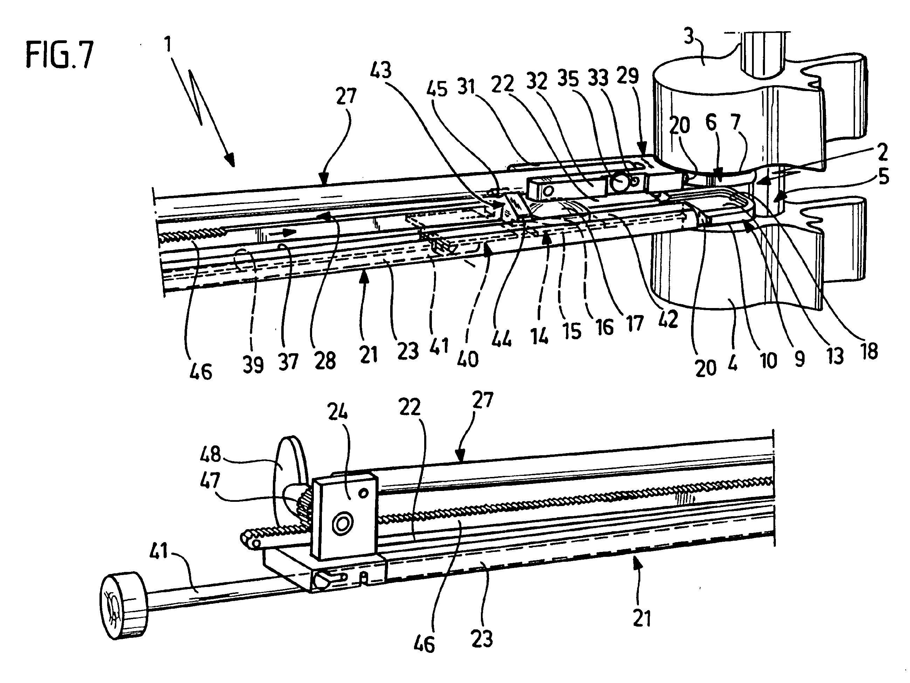

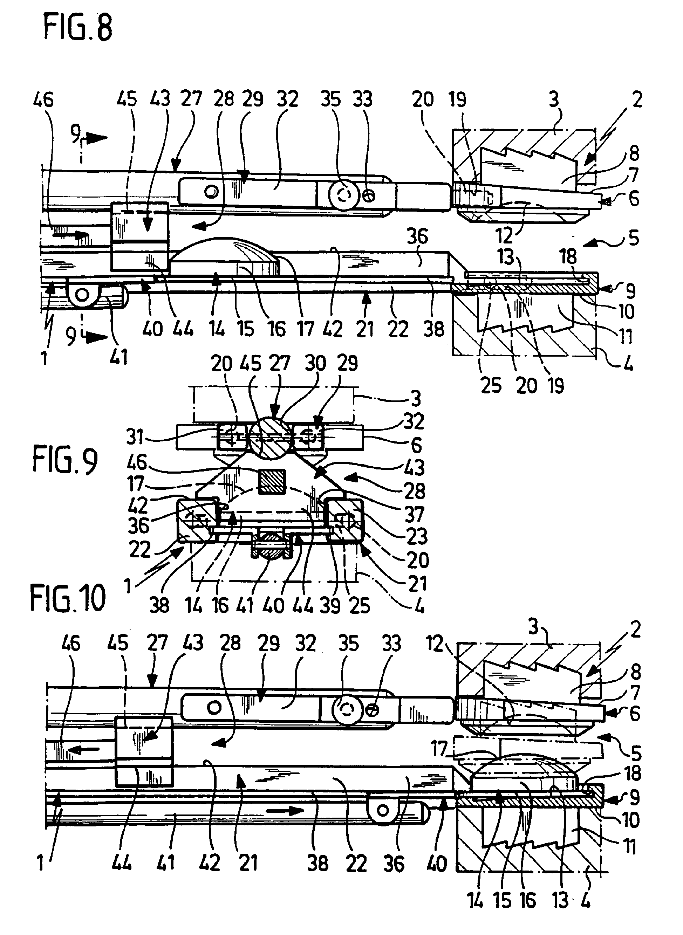

Durch Verdrehen des Zahnrads 47 und Vorschieben des Spreizelements 43 werden die Arme 21 und 27 auseinandergespreizt, dies führt zu einer Vergrößerung des gegenseitigen Abstandes von Oberteil 6 und Unterteil 9 und damit zu einer Aufweitung des Zwischenwirbelraums 5 (Figuren 7 bis 9). Die Aufweitung wird dabei so stark gewählt, daß mittels des Vorschubkörpers 40 das Gelenkelement 14 in die Vertiefung 13 des Unterteils 9 eingeschoben werden kann (Figur 10). Daran anschließend wird durch Zurückziehen des Spreizelements 43 der Abstand zwischen Oberteil 6 und Unterteil 9 wieder verringert, bis die Lagerflächen 12 und 17 ineinander eingreifen und die Teile des Zwischenwirbelimplantats 2 damit ihre Endposition erreicht haben (Figur 10, strichpunktierte Darstellung des Oberteils 6).

Durch Verdrehung der Schenkel 22 und 23 um ihre Längsachse wird der Eingriff des Riegelvorsprungs 25 in den Rücksprung 26 aufgehoben, und dann kann das Einsetzinstrument 1 von dem ordnungsgemäß eingesetzten Zwischenwirbelimplantat 2 abgezogen werden.

## Patentansprüche

1. Einsetzinstrument (1) für ein dreiteiliges Zwischenwirbelimplantat (2), welches ein an einem Wirbelkörper (3) anlegbares Oberteil (6), ein am benachbarten Wirbelkörper (4) anlegbares Unterteil (9) und ein dazwischen einsetzbares Gelenkelement (14) umfaßt, mit zwei an einem Ende gegeneinander schwenkbar gelagerten, nebeneinander angeordneten Armen (21, 27), die an ihrem freien Ende je eine Halteeinrichtung (20) für das Oberteil (6) beziehungsweise das Unterteil (9) des Zwischenwirbelimplantats (2) aufweisen, **dadurch gekennzeichnet, daß** in einem der Arme (21) eine Längsführung (38, 39) für das Gelenkelement (14) angeordnet ist.

2. Instrument nach Anspruch 1, **dadurch gekennzeichnet, daß** die Längsführung Längsnuten (38, 39) und das Gelenkelement (14) seitliche Vorsprünge (15) aufweist, die in die Längsnuten (38, 39) eingreifen.

3. Instrument nach Anspruch 2, **dadurch gekennzeichnet, daß** die Längsnuten (38, 39) der Längsführung einander gegenüberliegend sind, um einen in Längsrichtung des Armes (21) verlaufenden Aufnahmeraum (28) für das Gelenkelement (14) zu bilden.

4. Instrument nach einem der voranstehenden Ansprüche, **dadurch gekennzeichnet, daß** der die Längsführung (38, 39) aufweisende Arm (21) zwei im Abstand und parallel zueinander angeordnete, stabförmige Schenkel (22, 23) aufweist, die zwischen sich einen Aufnahmeraum (28) für das Gelenkelement (14) bilden, in welchem das Gelenkelement (14) in Längsrichtung des Armes (21) geführt ist.

5. Instrument nach einem der voranstehenden Ansprüche, **dadurch gekennzeichnet, daß** die Längsführung (38, 39) an ihrem der Schwenklagerung der Arme (21, 27) benachbarten Ende einen Einsetzbereich bildet, an dem das Gelenkelement (14) in die Längsführung (38, 39) einschiebbar ist.

6. Instrument nach einem der Ansprüche 2 bis 5, **dadurch gekennzeichnet, daß** das Unterteil (9) Nuten (18) aufweist, die mit den Nuten (38, 39) der Längsführung dann, wenn das Unterteil (9) an dem freien Ende des Armes (21) angebracht ist, derart fluchten, daß die seitlichen Ränder (15) des Gelenkelements (14) direkt von den Nuten (38, 39) der Längsführung in die Nuten (18) des Unterteils (9) beweglich sind.

7. Instrument nach einem der voranstehenden Ansprüche, **dadurch gekennzeichnet, daß** es einen in die Längsführung (38, 39) einsetzbaren, entlang dieser beweglichen Vorschubkörper (40) zum Vorschieben des Gelenkelements (14) und eine längliche Schubstange (41) umfaßt, die sich von dem Vorschubkörper (40) in Richtung der gelenkig gelagerten Enden der Arme (21, 27) erstreckt.

8. Instrument nach einem der voranstehenden Ansprüche, **dadurch gekennzeichnet, daß** die beiden Arme (21, 27) an ihrem freien Ende einander derart benachbart angeordnet und ausgebildet sind, daß die Halteeinrichtung (20) eines Armes neben der Halteeinrichtung des anderen Armes liegt.

9. Instrument nach einem voranstehenden Ansprüche, **dadurch gekennzeichnet, daß** die beiden Arme (21, 27) im Bereich ihrer Schwenklagerung einen Abstand voneinander haben, so daß die Arme (21, 27) in der Einsatzstellung, in welcher die freien Enden der Arme (21, 27) einander maximal angenähert sind, am gelagerten Ende einen größeren Abstand voneinander haben als am freien Ende.

10. Instrument nach Anspruch 9, **dadurch gekennzeichnet, daß** ein Spreizelement (43) vorgesehen ist, das sich an beiden Armen (21, 27) abstützt und längs der Arme (21, 27) in Richtung auf das freie Ende der Arme (21, 27) vorschiebbar ist und dabei die Arme (21, 27) um ihr Schwenklager (24) auseinander schwenkt.

11. Instrument nach Anspruch 10, **dadurch gekennzeichnet, daß** mindestens einer der beiden Arme (21, 27) eine Längsführung (42, 28; 27) für das Spreizelement (43) aufweist.

12. Instrument nach Anspruch 10 oder 11, **dadurch gekennzeichnet, daß** am Spreizelement (43) eine Vorschubstange (46) angeordnet ist.

13. Instrument nach Anspruch 12, **dadurch gekennzeichnet, daß** die Vorschubstange (46) als Zahnstange ausgebildet ist, die mit einem Antriebszahnrad (47) im Bereich der Schwenklagerung der Arme (21, 27) kämmt.

14. Instrument nach einem der voranstehenden Ansprüche, **dadurch gekennzeichnet, daß** die Haltevorrichtungen (20) Stifte sind, die in Öffnungen (19) des Oberteils (6) beziehungsweise des Unterteils (9) des Zwischenwirbelimplantats (2) eingreifen.

15. Instrument nach einem der voranstehenden Ansprüche, **dadurch gekennzeichnet, daß** die Haltevorrichtungen (20) zumindest an einem der Arme (27) um eine Schwenkachse verschwenkbar ist, die im Bereich des freien Endes des Armes (27) liegt und parallel zur Schwenkachse des Armes (27) verläuft, und daß die Haltevorrichtung nach dem Verschwenken um diese Schwenkachse in unterschiedlichen Winkelstellungen feststellbar ist.

16. Instrument nach Anspruch 15, **dadurch gekennzeichnet, daß** zur Feststellung der Haltevorrichtung (20) in verschiedenen Winkelstellungen ein Fixierstift (35) vorgesehen ist.

17. Instrument nach einem der voranstehenden Ansprüche, **dadurch gekennzeichnet, daß** mindestens eine Haltevorrichtung (20) eine lösbare Verriegelung (25, 26) zur Fixierung des von ihr gehaltenen Teils (9) des Implantats (2) aufweist.

18. Instrument nach Anspruch 17, **dadurch gekennzeichnet, daß** die Verriegelung der lösbaren Verriegelung (25, 26) durch die Verdrehung eines Riegels (25) um eine Drehachse erfolgt, die im wesentlichen parallel zur Längsachse des Armes (21) verläuft, an dem die Halteeinrichtung (20) angeordnet ist.

19. Instrument nach Anspruch 18, **dadurch gekennzeichnet, daß** wenigstens ein Teil (22, 23) des die Halteeinrichtung (20) tragenden Arms (21) um seine Längsachse verdrehbar ist, um den Riegel (25) zu drehen, so daß in einer Stellung der Riegel (25) des Arms (21) das gehaltene Teil (9) des zwischenwirbelimplantats (2) verriegelt und in einer anderen Winkelstellung freigibt.

20. Instrument nach Anspruch 19, **dadurch gekennzeichnet, daß** die Halteeinrichtung (20) einen in eine Aufnahmebohrung (19) am gehaltenen Teil (9) des Zwischenwirbelimplantats (2) eingreifenden Stift aufweist und der Riegel (25) seitlich von diesem Stift absteht, um in und außer Eingriff mit einem Rücksprung (26) in dem gehaltenen Teil (9) zu gelangen, um dieses zu verriegeln bzw. freizugeben.

21. Instrument nach einem der voranstehenden Ansprüche, **dadurch gekennzeichnet, daß** der die Längsführung (38, 39) aufweisende Arm (21) zwei parallel zueinander angeordnete Schenkel (22, 23) aufweist, die zwischen sich einen Aufnahmeraum (28) für das Gelenkelement (14) ausbilden, und daß der andere Arm (27) mittig zwischen diesen Schenkeln (22, 23) verläuft, so daß er mit seinem freien Ende zwischen die Schenkel (22, 23) bewegbar ist.

22. Instrument nach Anspruch 21, **dadurch gekennzeichnet, daß** ein zwischen den Armen (21, 27) angeordnetes, längs derselben verschiebliches Spreizelement (43) auf der Oberseite (42) der beiden Schenkel (22, 23) aufliegt, mit einem Vorsprung (44) zwischen die beiden Schenkel (22, 23) in den Aufnahmeraum (28) eingreift und an seiner Oberseite eine Vertiefung (45) zur Aufnahme des anderen Arms (27) aufweist.

23. Instrument nach Anspruch 21 oder 22, **dadurch gekennzeichnet, daß** die Schenkel (22, 23) des einen Armes (21) im Querschnitt rechteckig sind und der andere Arm (27) im Querschnitt kreisförmig ist.

24. Instrument nach einem der vorhergehenden Ansprüche, **dadurch gekennzeichnet, daß** ein Arm (21) ein Paar paralleler Schenkel (22, 23) und der zweite Arm (27) eine einzige, mittig zwischen den beiden Schenkeln (22, 23) des ersten Arms (21) liegende Stange aufweist, wobei die beiden Arme (21, 27) an einem Ende zueinander beabstandet sind, an welchen sie schwenkbar derart gelagert sind, daß die anderen freien Enden um die Schwenklagerung (24) aufeinander zu und voneinander weg beweglich sind.

25. Instrument nach Anspruch 24, **dadurch gekennzeichnet, daß** die Längsführungen Nuten (38, 39) an den Flanken der Schenkel (22, 23) des ersten Armes (21) aufweist, die einander zugewandt sind, und das Gelenkelement (14) seitliche Ränder (15) besitzt, die in die Nuten (38, 39) eingreifen.

26. Instrument nach Anspruch 25, **dadurch gekennzeichnet, daß** das Unterteil (9), das am freien Ende des ersten Arms (21) angebracht ist, Nuten (18) aufweist, die mit Nuten (38, 39) an den Schenkeln (22, 23) fluchten, wodurch die seitlichen Ränder (15) des Gelenkelements (14) entlang der Nuten (38, 39) der Schenkel in die Nuten (18) des Unterteils (9) beweglich sind.

27. Instrument nach Anspruch 26, **dadurch gekennzeichnet, daß** es einen Vorschubkörper (40), der ebenfalls in den Nuten (38, 39) der Schenkel angeordnet ist, und eine mit dem Vorschubkörper (40) verbundene Schubstange (41) aufweist, wobei der Vorschubkörper entlang der Nuten (38, 39) beweglich ist, um das Gelenkelement (14) entlang dieser in das Unterteil (9) vorzugschieben.

28. Instrument nach Anspruch 26 oder 27, **dadurch gekennzeichnet, daß** es ein Spreizelement (43) aufweist, das an den beiden Schenkeln (22, 23) des ersten Armes (21) und der einzelnen Stange des zweiten Armes (27) angreift und derart angeordnet und ausgebildet ist, daß es die Arme (21, 27) auseinander spreizt, wenn es entlang der Arme zu deren freien Enden hin bewegt wird.

29. Instrument nach einem der Ansprüche 24 bis 28, **dadurch gekennzeichnet, daß** sich der zweite Arm in der größten Annäherung des Oberteils (6) und des Unterteils (9) zueinander, wenn diese an den freien Enden der Arme (21, 27) angebracht sind, zwischen die beiden Schenkel (22, 23) des ersten Armes (21) bewegt.

## Claims

1. An insertion instrument (1) for a three piece intervertebral implant (2) that includes an upper part (6) which can be placed against a vertebra (3), a lower part (5) that can be placed against an adjacent vertebra (4) and a pivot element (14) that can be inserted between the upper and lower parts, the instrument having two arms (21, 27) disposed adjacent each other and supported pivotally relative to one another at one end, each arm including at its free end opposite said one end a retention device (20) for the upper part (6) and the lower part (9), respectively, of the intervertebral implant (2), **characterized in that** a longitudinal guide structure (38, 39) for the pivot element (14) is disposed in one of said arms (21).

2. An instrument according to claim 1, **characterized in that** the longitudinal guide structure includes longitudinal grooves (38, 39) and the pivot element (14) includes lateral protrusions (15) which engage said longitudinal grooves (38, 39).

3. An instrument according to claim 2, **characterized in that** the longitudinal grooves (38, 39) of the longitudinal guide structure face each other for forming a receiving chamber (28) for the pivot element (14) running along the longitudinal direction of that arm (21).

4. An instrument according to one of the preceding claims, **characterized in that** the arm (21) having the longitudinal guide structure (38, 39) comprises two legs (22, 23) disposed parallel to and spaced apart from one another, the legs between themselves forming a receiving chamber (28) in which the pivot element (14) is guided longitudinal along said arm (21).

5. An instrument according to one of the preceding claims, **characterized in that** the longitudinal guide structure (38, 39) forms near its end adjacent to the pivotal support of the arms (21, 27) an insertion region whereat the pivot element (14) can be inserted into the longitudinal guide structure (38, 39).

6. An instrument according to one of the claims 2 to 5, **characterized in that** the lower part (9) includes grooves (18) which are aligned with the grooves (38, 39) on the longitudinal guide structure when that lower part (9) is mounted on the free end of that arm (21) such that lateral edges (15) of the pivot element (14) can move directly from the grooves (38, 39) on the longitudinal guide structure into the grooves (18) on the lower part (9).

7. An instrument according to one of the preceding claims, **characterized in that** it includes a pusher (40) which is insertable in and slidable along the longitudinal guide structure (38, 39) for pushing the pivot element (14), the instrument further including an elongated rod (41) extending from said pusher (40) towards the pivotally supported ends of arms (21, 27).

8. An instrument according to one of the preceding claims, **characterized in that** the two arms (21, 27) are disposed adjacent each other at their free ends and constructed such that the retention device (20) on one of the arms lies next to the retention device on the other arm.

9. An instrument according to one of the preceding claims, **characterized in that** the two arms (21, 27), at their pivotally supported ends, are spaced from one another such that the arms (21, 27), in the insertion position in which the free ends of the arms (21, 27) are in their closest proximity to one another have a greater spacing from one another at their pivotally supported ends than at their free ends.

10. An instrument according to claim 9, **characterized in that** a spreader element (43) is provided which is supported on the arms (21, 27) the spreader element (43) being movable along the arms in the direction toward the free ends of the arms (21, 27) to pivot the two' arms (21, 27) about their pivotal support (24) away from each other.

11. An instrument according to claim 10, **characterized in that** at least one of the two arms (21, 27) has a longitudinal guide (42, 28; 27) for the spreader element (43), and includes an elongated feed rod connected to the spreader element.

12. An instrument according to claim 10 or 11, **characterized in that** on the spreader element (43) there is provided a feed rod (46).

13. An instrument according to claim 2, **characterized in that** the feed rod (46) is formed by a rack which meshes with a driving gear wheel (47) in the region of the pivotal support of the arms (21, 27).

14. An instrument according to on of the preceding claims, **characterized in that** the retention devices (20) are pins which engage bores (15) in the upper (6) and lower (9) parts, respectively, of the intervertebral implant (2).

15. An instrument according to one of the preceding claims, **characterized in that** the retention device (20) on at least one of the arms (27) is rotatable about an axis that is located in the region of the free end of that arm (27) and which extends parallel to the pivot axis of that arm (27), and wherein the retention device, after being pivoted about this axis, can be locked in different angular positions.

16. An instrument according to claim 15, **characterized in that** a fixation pin (35) is provided for locking the retention device (20) at different angular positions.

17. An instrument according to one of the preceding claims, **characterized in that** at least one of the retention devices (20) has a releasable locking means (25, 26) for locking the part (9) of the implant (8) being held by said retention device (20).

18. An instrument according to claim 17, **characterized in that** locking of the releasable locking means (25, 26) is effected by rotating a locking bar (25) about an axis of rotation, which axis extends substantially parallel to the longitudinal axis of the arm (21) on which the retention device (20) is arranged.

19. An instrument according to claims 18, **characterized in that** at least a portion (22, 23) of the arm (21) carrying the retention device (20) is rotatable about its longitudinal axis for rotating the locking bar such that in one position the locking bar (25) of the arm (21) locks the connected part (9) of the implant (2) and in another angular position, releases it.

20. An instrument according to claim 19, **characterized in that** the retention device (20) has a pin which engages a receiving bore (19) on the connected implant part (9) and the locking bar (25) protrudes laterally from this pin to engage or disengage a notch (26) on the connected implant part (9) to lock or release it, respectively.

21. An instrument according to one of the preceding claims, **characterized in that** the arm (21) having the longitudinal guide structure (38, 39) comprises two parallel legs (22, 23) which form between them a receiving chamber (28) for receiving the pivot element (24) and wherein the other arm (27) extends centrally between the legs (22, 23) so that its free end can dip between the parallel legs.

22. An instrument according to claim 21, **characterized in that** a spreader element (43) is disposed between the two arms and displaceable along them, said spreader element (43) resting on the surface (42) of the two legs (22, 23) and having a protrusion (44) which extends between the two legs into the receiving chamber and an indentation (45) on its top for receiving the other arm (27).

23. An instrument according to claim 21 or 22, **characterized in that** the two parallel legs (22, 23) of one of the arms (21) are rectangular in cross section, and the other arm (27) is circular in cross section.

24. An instrument according to one of the preceding claims, **characterized in that** a first of said arms (21) comprises a pair a parallel legs (22, 23) and the second arm (27)comprises a single rod located centrally between the two legs (22, 23) of the first arm (26), the two arms spaced apart at one end where they are pivotally supported, such that the other ends, which are said free ends, are moveable about said pivotal support (24), towards and away from each other.

25. An instrument according to claim 24, **characterized in that** said longitudinal guide structure grooves (38, 39) on the sides of the legs (22, 23) of the first arm (21) which face each other, and the pivot element (14) has lateral edges (15) which engage said grooves (38, 39).

26. An instrument according to claim 25, **characterized in that** the lower part (9) which is mounted on the free end of said first arm (21) has grooves (18) that are aligned with the grooves (38, 39) on the legs (22, 23), whereby the lateral edges (15) of the pivot element (14) are movable along the grooves (38, 39) of the legs and then into the grooves (18) of the lower part (9).

27. An instrument according to claim 26, **characterized in that** it includes a pusher (40), also mounted in the grooves (38, 39) of the legs and a rod (41) connected to the pusher (40), the pusher being movable along the grooves (38, 39) to push the pivot element (14) therealong and into the lower part (19).

28. An instrument according to claim 26 or 27, **characterized in that** it includes a spreader element (40) engaging the two legs (22, 23) of the first arm (21) and the single rod of the second arm (27) and positioned and shaped such that when moved along the arms toward the free ends, it spreads the arms apart from each other.

29. An instrument according to one of the claims 24 to 28 **characterized in that** in the closest proximity of the upper and lower parts (6, 9) to each other, when mounted on the free ends of the arms (21, 27) the second arm moves between the two legs (22, 23) of the first arm (21).

## Revendications

1. Instrument d'insertion (1) pour un implant intervertébral (2) en trois parties, comprenant une partie supérieure (6) pouvant être placée sur un corps vertébral (3), une partie inférieure (9) pouvant être placée sur le corps vertébral adjacent (4) et un élément d'articulation (14) pouvant être inséré entre les deux, dans lequel deux bras (21, 27) disposés l'un à côté de l'autre et montés à une extrémité de manière à pivoter l'un par rapport à l'autre présentent chacun un dispositif de support (20) à leur extrémité libre pour la partie supérieure (6) ou la partie inférieure (9) de l'implant intervertébral (2),
**caractérisé en ce qu'**
une glissière longitudinale (38, 39) pour l'élément d'articulation (14) est disposée dans un des bras (21).

2. Instrument selon la revendication 1,
**caractérisé en ce que**
la glissière longitudinale présente des rainures longitudinales (38, 39) et l'élément d'articulation (14) présente des saillies latérales (15) qui s'engagent dans les rainures longitudinales (38, 39).

3. Instrument selon la revendication 2,
**caractérisé en ce que**
les rainures longitudinales (38, 39) de la glissière longitudinale se font face de manière à former pour l'élément d'articulation (14) un espace de réception (28) s'étendant dans la direction longitudinale du bras (21).

4. Instrument selon l'une des revendications précédentes,
**caractérisé en ce que**
le bras (21) présentant la glissière longitudinale (38, 39) présente deux branches (22, 23) en forme de barre écartées parallèlement l'une de l'autre et formant entre elles pour l'élément d'articulation (14) un espace de réception (28) guidant l'élément d'articulation (14) dans la direction longitudinale du bras (21).

5. Instrument selon l'une des revendications précédentes,
**caractérisé en ce que**
la glissière longitudinale (38, 39) forme, à son extrémité adjacente au point de pivotement des bras (21, 27), une zone d'insertion contre laquelle peut être poussé l'élément d'articulation (14) dans la glissière longitudinale (38, 39).

6. Instrument selon l'une des revendications 2 à 5,
**caractérisé en ce que**
la partie inférieure (9) présente des rainures (18) qui lorsque la partie inférieure (9) est appliquée contre l'extrémité libre du bras (21) s'alignent avec les rainures (38, 39) de la glissière longitudinale de telle sorte que les bords latéraux (15) de l'élément d'articulation (14) puisent directement être déplacés des rainures (38, 39) de la glissière longitudinale dans les rainures (18) de la partie inférieure (9).

7. Instrument selon l'une des revendications précédentes,
**caractérisé en ce qu'**
un corps d'avancement (40) est inséré dans la glissière longitudinale (38, 39) et mobile le long de celle-ci pour faire avancer l'élément d'articulation (14) ainsi qu'une tige de poussée longitudinale (41) qui s'étend du corps d'avancement (40) dans la direction des extrémités des bras (21, 27) montées de manière articulée.

8. Instrument selon l'une des revendications précédentes,
**caractérisé en ce que**
les deux bras (21, 27) ont leurs extrémités libres voisines l'une de l'autre et configurées de telle sorte que le dispositif de support (20) d'un bras se trouve contre le dispositif de support de l'autre bras.

9. Instrument selon l'une des revendications précédentes,
**caractérisé en ce que**
les deux bras (21, 27) sont distants l'un de l'autre au niveau de leur point de pivotement de telle sorte que les bras (21, 27), dans leur position d'insertion où les extrémités libres des bras (21, 27) sont rapprochées l'une de l'autre au maximum, présentent à l'extrémité mise en place un écart plus important qu'à l'extrémité libre.

10. Instrument selon la revendication 9,
**caractérisé en ce qu'**
un élément d'écartement (43) s'appuie sur les deux bras (21, 27) et peut être avancé le long des bras (21, 27) dans la direction de l'extrémité libre des bras (21, 27) pour faire pivoter les bras (21, 27) l'un par rapport à l'autre autour de leur palier de pivotement (24).

11. Instrument selon la revendication 10,
**caractérisé en ce qu'**
au moins un des deux bras (21, 27) présente une glissière longitudinale (42, 28 ; 27) pour l'élément d'écartement (43).

12. Instrument selon la revendication 10 ou 11,
**caractérisé en ce qu'**
une tige d'avancement (46) est disposée sur l'élément d'écartement (43).

13. Instrument selon la revendication 12,
**caractérisé en ce que**
la tige d'avancement (46) a la forme d'une crémaillère qui s'engrène avec un engrenage d'entraînement (47) au niveau du point de pivotement des bras (21, 27).

14. Instrument selon l'une des revendications précédentes,
**caractérisé en ce que**
les dispositifs de support (20) sont des goupilles qui s'engagent dans des ouvertures (19) de la partie supérieure (6) ou de la partie inférieure (9) de l'implant intervertébral (2).

15. Instrument selon l'une des revendications précédentes,
**caractérisé en ce que**
le dispositif de support (20) peut pivoter au moins sur un des bras (27) autour d'un axe de pivotement situé au niveau de l'extrémité libre du bras (27) et parallèle à l'axe de pivotement du bras (27), et le dispositif de support, après avoir pivoté autour de cet axe de pivotement, peut être fixé dans différentes positions angulaires.

16. Instrument selon la revendication 15,
**caractérisé en ce qu'**
une goupille de fixation (35) est prévue pour fixer le dispositif de support (20) dans différentes positions angulaires.

17. Instrument selon l'une des revendications précédentes,
**caractérisé en ce qu'**
au moins un dispositif de support (20) présente un moyen de verrouillage desserrable (25, 26) pour fixer la partie (9) de l'implant (2) maintenue par celui-ci.

18. Instrument selon la revendication 17,
**caractérisé en ce que**
le verrouillage du moyen de verrouillage desserrable (25, 26) s'effectue en faisant tourner un verrou (25) autour d'un axe de rotation sensiblement parallèle à l'axe longitudinal du bras (21) portant le dispositif de support (20).

19. Instrument selon la revendication 18,
**caractérisé en ce qu'**
au moins une partie (22, 23) du bras (21) portant le dispositif de support (20) peut être tournée autour de son axe longitudinal pour faire tourner le verrou (25) de telle sorte que, dans une position, le verrou (25) du bras (21) verrouille la partie maintenue (9) de l'implant intervertébral (2) et la libère dans une autre position angulaire.

20. Instrument selon la revendication 19,
**caractérisé en ce que**
le dispositif de support (20) présente une goupille s'engageant dans un alésage de réception (19) sur la partie maintenue (9) de l'implant intervertébral (2) et le verrou (25) dépasse latéralement de cette goupille pour parvenir dans la partie maintenue (9) avec un rappel (26) en prise ou hors prise, et verrouiller ou libérer celle-ci.

21. Instrument selon l'une des revendications précédentes,
**caractérisé en ce que**
le bras (21) présentant la glissière longitudinale (38, 39) présente deux branches (22, 23) parallèles formant entre elles un espace de réception (28) pour l'élément d'articulation (14), et l'autre bras (27) s'étend au milieu entre ces branches (22, 23) pour pouvoir se déplacer avec son extrémité libre entre les branches (22, 23).

22. Instrument selon la revendication 21,
**caractérisé en ce qu'**
un élément d'écartement (43) mobile entre les bras (21, 27) le long de ceux-ci repose sur la face supérieure (42) des deux branches (22, 23), s'engage avec une saillie (44) dans l'espace de réception (28) entre les deux branches (22, 23) et sa face supérieure présente un évidement (45) pour recevoir l'autre bras (27).

23. Instrument selon la revendication 21 ou 22,
**caractérisé en ce que**
les branches (22, 23) du premier bras (21) ont une section transversale rectangulaire et celles de l'autre bras (27) ont une section transversale circulaire.

24. Instrument selon l'une des revendications précédentes,
**caractérisé en ce qu'**
un bras (21) présente une paire de branches parallèles (22, 23) et le deuxième bras (27) présente une seule tige située au milieu entre les deux branches (22, 23) du premier bras (21), et les deux bras (21, 27) à une extrémité où ils sont montés pivotants sont écartés pour que les autres extrémités libres se déplacent autour du point de pivotement (24), l'une vers l'autre et l'une à distance de l'autre.

25. Instrument selon la revendication 24,
**caractérisé en ce que**
les glissières longitudinales présentent sur les flancs des branches (22, 23) du premier bras (21), des rainures (38, 39) en regard l'une de l'autre et l'élément d'articulation (14) comporte des bords latéraux (15) qui s'engagent dans les rainures (38, 39).

26. Instrument selon la revendication 25,
**caractérisé en ce que**
la partie inférieure (9) appliquée à l'extrémité libre du premier bras (21) présente des rainures (18) qui s'alignent avec des rainures (38, 39) sur les branches (22, 23), ce qui permet aux bords latéraux (15) de l'élément d'articulation (14) le long des rainures (38, 39) des branches d'être mobiles dans les rainures (18) de la partie inférieure (9).

27. Instrument selon la revendication 26,
**caractérisé en ce qu'**
un organe de poussée (40) qui est également disposé dans les rainures (38, 39) des branches ainsi qu'une tige de poussée (41) reliée au corps d'avancement (40), l'organe de poussée étant mobile le long des rainures (38, 39) pour pousser l'élément d'articulation (14) le long de celles-ci dans la partie inférieure (9).

28. Instrument selon la revendication 26 ou 27,
**caractérisé en ce qu'**
un élément d'écartement (43) agit sur les deux branches (22, 23) du premier bras (21) et sur la seule tige du deuxième bras (27) et est disposé et configuré pour écarter les bras (21, 27) l'un de l'autre lorsqu'il est déplacé le long des bras vers leurs extrémités libres.

29. Instrument selon l'une des revendications 24 à 28,
**caractérisé en ce que**
lorsque la partie supérieure (6) et la partie inférieure (9) sont le plus rapprochées l'une de l'autre, quand celles-ci sont appliquées contre les extrémités libres des bras (21, 27), le deuxième bras se déplace entre les deux branches (22, 23) du premier bras (21).
